(19) Europäisches Patentamt
European Patent Office
Office européen des brevets

(11) Veröffentlichungsnummer: **0 128 274**
**A1**

(12) # EUROPÄISCHE PATENTANMELDUNG

(21) Anmeldenummer: **84102917.6**

(22) Anmeldetag: **16.03.84**

(51) Int. Cl.[3]: **C 07 D 239/42**, C 07 D 239/46, C 07 D 251/16, C 07 D 251/18, C 07 D 251/22, C 07 D 251/46, C 07 D 251/52, A 01 N 47/36

(30) Priorität: **16.06.83 DE 3322280**

(43) Veröffentlichungstag der Anmeldung: **19.12.84 Patentblatt 84/51**

(84) Benannte Vertragsstaaten: **BE CH DE FR GB IT LI LU NL SE**

(71) Anmelder: **SCHERING AKTIENGESELLSCHAFT Berlin und Bergkamen, Müllerstrasse 170/178 Postfach 65 03 11, D-1000 Berlin 65 (DE)**

(72) Erfinder: **Westermann, Jürgen, Dr., Elberfelderstrasse 19, D-1000 Berlin 21 (DE)**
Erfinder: **Boroschewski, Gerhard, Dr., Wilkestrasse 15, D-1000 Berlin 27 (DE)**
Erfinder: **Eder, Ulrich, Dr., Zeitinger Strasse 17, D-1000 Berlin 28 (DE)**
Erfinder: **Arndt, Friedrich, Dr., Mühlenfeldstrasse 10, D-1000 Berlin 28 (DE)**
Erfinder: **Krähmer, Hansjörg, Dr., Fürstendamm 10A, D-1000 Berlin 28 (DE)**
Erfinder: **Kötter, Clemens, Dr., Laurinsteig 26, D-1000 Berlin 28 (DE)**

(54) **Substituierte Sulfonylharnstoffe, Verfahren zur Herstellung dieser Verbindungen sowie diese enthaltende Mittel mit herbizider und pflanzenwuchsregulatorischer Wirkung.**

(57) Die Erfindung betrifft neue substituierte Sulfonylharnstoffe der allgemeinen Formel

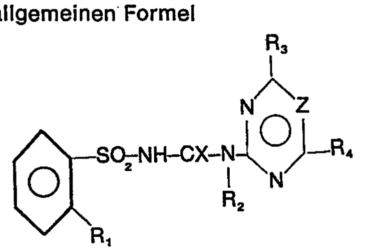

(I)

in der

$R_1$ Chlor, $-COOR_5$, $-S(O)_n-R_6$ oder 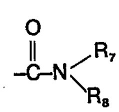 ,

$R_2$ die Gruppen 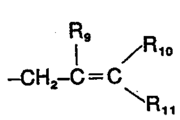 oder

$-CH_2-C\equiv C-R_{11}$,

$R_3$ Wasserstoff, $C_1$–$C_4$-Alkyl, $C_1$–$C_4$-Alkoxy, $C_1$–$C_4$-Alkylthio, Halogen, Halogen-$C_1$–$C_4$-alkyl, Halogen-$C_1$–$C_4$-alkoxy, Di-$C_1$–$C_3$-alkyl-amino, $C_1$–$C_3$-Alkyl-amino oder $C_1$–$C_3$-Alkoxy-$C_1$–$C_3$-alkoxy,

$R_4$ Wasserstoff, $C_1$–$C_4$-Alkyl, $C_1$–$C_4$-Alkoxy, $C_1$–$C_4$-Alkylthio, Halogen, Halogen-$C_1$–$C_4$-alkyl, Halogen-$C_1$–$C_4$-alkoxy, Di-$C_1$–$C_3$-alkyl-amino, $C_1$–$C_3$-Alkyl-amino oder $C_1$–$C_3$-Alkoxy-$C_1$–$C_3$-alkoxy,

Z $-CH=$ oder $-N=$,

$R_5$ $C_1$–$C_8$-Alkyl, $C_3$–$C_8$-Cycloalkyl, $C_1$–$C_3$-Alkoxy-$C_1$–$C_3$-alkyl, Phenyl, substituiertes Phenyl, Benzyl oder substituiertes Benzyl,

$R_6$ $C_1$–$C_6$-Alkyl oder Phenyl,

$R_7$ $C_1$–$C_4$-Alkyl,

$R_8$ $C_1$–$C_4$-Alkyl,

$R_7$ und $R_8$ $C_3$–$C_6$-Cycloalkyl, Morpholinyl, Pyrrolidinyl, Piperidyl oder Piperazinyl,

$R_9$ Wasserstoff, Chlor, Fluor oder $C_1$–$C_3$-Alkyl,

$R_{10}$ Wasserstoff, Chlor, Fluor, Trifluormethyl oder $C_1$–$C_3$-Alkyl,

$R_{11}$ Wasserstoff, Chlor, Fluor, Cyano, $C_1$–$C_4$-Alkyl oder Phenyl,

X Sauerstoff oder Schwefel und

n 0, 1 oder 2 bedeuten.

Verfahren zur Herstellung dieser Verbindungen sowie diese enthaltende Mittel mit herbizider und pflanzenwuchsregulierender Wirkung.

EP 0 128 274 A1

Die Erfindung betrifft neue substituierte Sulfonylharnstoffe, Verfahren zur Herstellung dieser Verbindungen sowie diese enthaltende Mittel mit herbizider und pflanzenwuchsregulierender Wirkung.

Sulfonamide mit herbizider Wirkung sind bereits bekannt (EP-PS 0001515).

Aufgabe der vorliegenden Erfindung ist die Entwicklung neuer Wirkstoffe mit überlegenen Eigenschaften.

Diese Aufgabe wird erfindungsgemäß gelöst durch neue substituierte Sulfonylharnstoffe der allgemeinen Formel

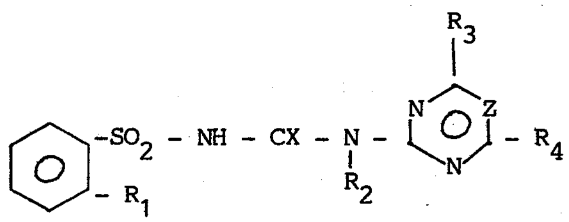

I

in der

$R_1$ Chlor, $-COOR_5$, $-S(O)_n-R_6$ oder 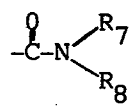 ,

$R_2$ die Gruppen 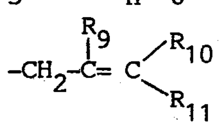 oder $-CH_2-C \equiv C-R_{11}$ ,

$R_3$ Wasserstoff, $C_1$-$C_4$-Alkyl, $C_1$-$C_4$-Alkoxy, $C_1$-$C_4$-Alkylthio, Halogen, Halogen-$C_1$-$C_4$-alkyl, Halogen-$C_1$-$C_4$-alkoxy, Di-$C_1$-$C_3$-alkyl-amino, $C_1$-$C_3$-Alkyl-amino oder $C_1$-$C_3$-Alkoxy-$C_1$-$C_3$-alkoxy,

$R_4$ Wasserstoff, $C_1$-$C_4$-Alkyl, $C_1$-$C_4$-Alkoxy, $C_1$-$C_4$-Alkylthio, Halogen, Halogen-$C_1$-$C_4$-alkyl, Halogen-$C_1$-$C_4$-alkoxy, Di-$C_1$-$C_3$-alkyl-amino, oder $C_1$-$C_3$-Alkoxy-$C_1$-$C_3$-alkoxy,

Z -CH= oder -N=,

$R_5$ $C_1$-$C_8$-Alkyl, $C_3$-$C_8$-Cycloalkyl, $C_1$-$C_3$-Alkoxy-$C_1$-$C_3$-alkyl, Phenyl, substituiertes Phenyl, Benzyl oder substituiertes Benzyl,



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

$R_6$ $C_1$-$C_6$-Alkyl oder Phenyl,
$R_7$ $C_1$-$C_4$-Alkyl,
$R_8$ $C_1$-$C_4$-Alkyl,
$R_7$ und $R_8$ $C_3$-$C_8$-Cycloalkyl, Morpholinyl, Pyrrolidinyl, Piperidyl oder Piperazinyl,
$R_9$ Wasserstoff, Chlor, Fluor oder $C_1$-$C_3$-Alkyl,
$R_{10}$ Wasserstoff, Chlor, Fluor, Trifluormethyl oder $C_1$-$C_3$-Alkyl,
$R_{11}$ Wasserstoff, Chlor, Fluor, Cyano, $C_1$-$C_4$-Alkyl oder Phenyl,
X Sauerstoff oder Schwefel und
n 0, 1 oder 2 bedeuten.

Die erfindungsgemäßen substituierten Sulfonylharnstoffe weisen eine ausgezeichnete herbizide Wirkung gegen schwer bekämpfbare zweikeimblättrige und grasartige, annuelle und perennierende Unkräuter auf.

Bekämpft werden zum Beispiel Stellaria, Abutilon, Matricaria, Viola, Centaurea, Amaranthus, Fagopyrum, Helianthus, Brassica, Sesbania, Euphorbia, Datura, Avena fatua, Alopecurus myosuroides, Mentha arvensis, Cirsium arvense, Convolvulus arvensis, Sorghum halepense, Cyperus esculentus, Poa annua, Bromus tectorum und Agropyron repens.

Die Anwendung kann im Vorsaat-, Vorauflauf- oder Nachauflaufverfahren erfolgen.

Erfolgt die Behandlung vor dem Auflaufen der Unkräuter auf unbewachsenem Boden, so laufen die keimenden Pflanzen auf und entwickeln sich bis zum Keimblattstadium, dann erfolgt jedoch ein völliger Wachstumsstop und schließlich nach 3 bis 5 Wochen sterben die Pflanzen ab.

Bei Anwendung der Wirkstoffe im Nachauflaufverfahren tritt rasch nach der Behandlung ein Stop des Wachstums ein. Die



Schering Aktiengesellschaft, Postfach 65 03 11, D-1000 Berlin 65 · Für Besucher: Berlin-Wedding, Müllerstraße 170-178 · Telegramme: Scheringchemie Berlin
Dr. Heinz Hannse Horst Kramp Dr Klaus Pohle, Dr Horst Witzel Vorsitzender des Aufsichtsrats: Hans-Jürgen Hamann
Berlin und Bergkamen Handelsregister: AG Charlottenburg 93 HRB 283 und AG Kamen HRB 0061 Berliner Commerzbank AG, Berlin, Konto-Nr. Berliner Handels- und Frankfurter Bank, Berlin, Konto-Nr. 70045224, Bankleitzahl 100 202 00 Deutsche Bank Berlin AG, Konto-Nr. Postscheckamt Berlin West, Konto-Nr. 1175-101, Bankleitzahl 100 100 10

Unkräuter bleiben in diesem Wachstumsstadium stehen oder sterben nach längerer Zeit ganz ab.

Die Wirkstoffe besitzen vorteilhafterweise schon in sehr niedrigen Aufwandmengen eine herbizide Wirkung. Vorzugsweise liegen die Aufwandmengen je ha von 0,010 bis 5,0 kg Wirkstoff/ha.

Die erfindungsgemäßen Wirkstoffe lassen sich in der vorbeschriebenen Weise auch zur totalen Unkrautbekämpfung einsetzen.
Überraschenderweise verhalten sich aber wichtige Großkulturen, wie zum Beispiel Mais, Gerste, Weizen, Reis, Baumwolle und Soja,resistent, so daß die erfindungsgemäßen Verbindungen mit großem Vorteil zur selektiven Bekämpfung monokotyler und dikotyler, annueller und perennierender Unkräuter eingesetzt werden können, die mit dem heutigen Stand der Technik nur schwer oder auch gar nicht erfaßbar sind.

Die erfindungsgemäßen Verbindungen eignen sich außerdem zur Beeinflussung des vegetativen und generativen Wachstums bei Leguminosen, wie zum Beispiel Soja, können aber auch bei Gramineen eingesetzt werden. So lassen sie sich zum Beispiel aufgrund retardierender Effekte zur Steigerung von Zuckergehalten bei Zuckerrohr verwenden.

Aufgrund dieser Eigenschaften lassen sich die erfindungsgemäßen Verbindungen in die Klasse der Pflanzenwachstumsregulatoren einstufen, die sich durch folgende Anwendungsmöglichkeiten auszeichnen:

-4-

Postanschrift: Schering Aktiengesellschaft, Postfach 65 03 11, D-1000 Berlin 65 · Für Besucher: Berlin-Wedding, Müllerstraße 170-178 Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

Hemmung des vegetativen Wachstums bei holzigen und krautigen Pflanzen zum Beispiel an Straßenrändern, Gleisanlagen u.a., um ein zu üppiges Wachstum zu unterbinden. Wuchshemmung beim Getreide, um das Lagern oder Umknicken zu unterbinden, bei Baumwolle zur Ertragserhöhung.

Beeinflussung der Verzweigung von vegetativen und generativen Organen bei Zier- und Kulturpflanzen zur Vermehrung des Blütenansatzes oder bei Tabak und Tomate zur Hemmung von Seitentrieben.

Verbesserung der Fruchtqualität, zum Beispiel eine Zuckergehaltssteigerung beim Zuckerrohr, bei Zuckerrüben oder bei Obst, und eine gleichmäßigere Reife des Erntegutes, die zu höheren Erträgen führt.

Erhöhung der Widerstandskraft gegen klimatische Einflüsse wie Kälte und Trockenheit.

Beeinflussung des Latexflusses bei Gummipflanzen.

Ausbildung parthenokarper Früchte, Pollensterilität und Geschlechtsbeeinflussung sind ebenfalls Anwendungsmöglichkeiten.

Kontrolle der Keimung von Samen oder des Austriebs von Knospen.

Entlaubung oder Beeinflussung des Fruchtfalles zur Ernteerleichterung.

Die erfindungsgemäßen Stoffe entfalten diese Effekte sowohl bei der Vorals auch bei der Nachauflaufbehandlung.

Die Aufwandmengen für die pflanzenwuchsregulierende Wirkung betragen je nach Anwendungsziel im allgemeinen von 0,005 bis 5 kg Wirkstoff/ha, es können jedoch gegebenenfalls auch höhere Aufwandmengen eingesetzt werden.

-5-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Heinz-Hartmut Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG Berlin, Konto-Nr. 108700, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

Die Anwendungszeit richtet sich nach dem Anwendungsziel und den klimatischen Bedingungen.

Von den erfindungsgemäßen Verbindungen sind insbesondere die folgenden zu nennen, die sich durch eine überlegene herbizide und pflanzenwuchsregulierende Wirkung gegenüber dem Stand der Technik auszeichnen:

**1-Allyl-1-(4,6-dimethyl-2-pyrimidinyl)-3-(2-methoxycarbonyl-phenylsulfonyl)-harnstoff,**

**1-(4,6-Dimethyl-2-pyrimidinyl)-3-(2-methoxycarbonyl-phenylsulfonyl)-1-propargyl-harnstoff,**

**1-Allyl-1-(4,6-dimethyl-2-pyrimidinyl)-3-(2-äthoxycarbonyl-phenylsulfonyl)-harnstoff,**

**1-(4,6-Dimethyl-2-pyrimidinyl)-3-(2-äthoxycarbonyl-phenylsulfonyl)-1-propargyl-harnstoff,**

**1-Allyl-1-(4,6-dimethoxy-1,3,5-triazin-2-yl)-3-(2-äthoxycarbonyl-phenylsulfonyl)-harnstoff,**

**1-Allyl-1-(4-methoxy-6-methyl-1,3,5-triazin-2-yl)-3-(2-methoxycarbonyl-phenylsulfonyl)-harnstoff,**

**1-Allyl-1-(4-methoxy-6-methyl-1,3,5-triazin-2-yl)-3-(2-äthoxycarbonyl-phenylsulfonyl)-harnstoff.**

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden.

-6-

**Postanschrift: Schering Aktiengesellschaft, Postfach 65 03 11, D-1000 Berlin 65** · Fur Besucher Berlin-Wedding, Mullerstraße 170-178 · Telegramme: Scheringchemie Berlin

Vorstand Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft Berlin und Bergkamen · Handelsregister AG Charlottenburg 93 HRB 283 und AG Kamen HRB 0061 · Berliner Commerzbank AG, Berlin, Konto-Nr. 108700600 Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008 Bankleitzahl 100 700 00 · Postscheckamt Berlin West, Konto-Nr. 11 75-101, Bankleitzahl 100 100 10

Sofern eine Verbreiterung des Wirkungsspektrums beabsichtigt ist, können auch andere Biozide zugesetzt werden. Beispielsweise eignen sich als herbizid wirksame Mischungspartner diejenigen Wirkstoffe, die in Weed Abstracts, Vol. 31, No. 7, 1982 unter dem Titel "Lists of common names and abbreviations employed for currently used herbicides and plant growth regulators in weed abstracts" aufgeführt sind. Außerdem können auch nicht phytotoxische Mittel angewendet werden, die mit Herbiziden und/oder Wuchsregulatoren eine synergistische Wirkungssteigerung ergeben können, wie unter anderem Netzmittel, Emulgatoren, Lösungsmittel und ölige Zusätze.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acyl-phosphatidylethanolaminen, Phosphatidylinosit, Phosphatidylserin, Lysolecithin und Phosphatidylglycerol.

Zweckmäßig werden die gekennzeichneten Wirkstoffe oder deren Mischung in Form von Zubereitungen, wie Pulvern, Streumitteln, Granulaten, Lösungen, Emulsionen oder suspensionen, unter Zusatz von flüssigen und/oder festen Trägerstoffen beziehungsweise Verdünnungsmitteln und gegebenenfalls Netz-Haft-, Emulgier- und/oder Dispergierhilfsmitteln angewandt.

Geeignete flüssige Trägerstoffe sind zum Beispiel Wasser, aliphatische und aromatische Kohlenwasserstoffe, wie Benzol, Toluol, Xylol, Cylohexanon, Isophoron, Dimethylsulfoxyd, Dimethylformamid, weiterhin Mineralölfraktionen und Pflanzenöle.

Als feste Trägerstoffe eignen sich Mineralerden, zum Beispiel Tonsil, Silicagel, Talkum, Kaolin, Attapulgit, Kalkstein, Kieselsäure und pflanzliche Produkte, zum Beispiel Mehle.

-7-

An oberflächenaktiven Stoffen sind zu nennen: zum Beispiel Calciumligninsulfonat, Polyoxyäthylenalkylphenyl-äther, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Sofern die Wirkstoffe zur Saatgutbeizung Verwendung finden sollen, können auch Farbstoffe zugemischt werden, um dem gebeizten Saatgut eine deutlich sichtbare Färbung zu geben.

Der Anteil des bzw. der Wirkstoffe(s) in den verschiedenen Zubereitungen kann in weiten Grenzen variieren. Beispielsweise enthalten die Mittel etwa 10 bis 90 Gewichtsprozent Wirkstoffe, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen von etwa 100 bis 1000 Liter/ha. Eine Anwendung der Mittel im sogenannten "Low-Volume- oder Ultra-low-volume-Verfahren" ist ebenso möglich wie ihre Applikation in Form von sogenannten Mikrogranulaten.

Zur Herstellung der Zubereitungen werden zum Beispiel die folgenden Bestandteile eingesetzt:

A. Spritzpulver

a) 40 Gewichtsprozent Wirksotff
25 Gewichtsprozent Tonmineralien
20 Gewichtsprozent Kieselsäure
10 Gewichtsprozent Zellpech
5 Gewichtsprozent oberflächenaktive Stoffe auf der Basis einer Mischung des Calciumsalzes der Ligninsulfonsäure mit Alkylphenolpolyglykoläthern



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

b) 25 Gewichtsprozent Wirkstoff
60 Gewichtsprozent Kaolin
10 Gewichtsprozent Kieselsäure
5 Gewichtsprozent oberflächenaktive Stoffe auf Basis des Natriumsalzes des N-Methyl-N-oleyl-taurins und des Calciumsalzes der Ligninsulfonsäure.

c) 10 Gewichtsprozent Wirkstoff
60 Gewichtsprozent Tonmineralien
15 Gewichtsprozent Kieselsäure
10 Gewichtsprozent Zellpech
5 Gewichtsprozent oberflächenaktive Stoffe auf Basis des Natriumsalzes des N-Methyl-N-oleyl-taurins und des Calciumsalzes der Ligninsulfonsäure

B. Paste

45 Gewichtsprozent Wirkstoff
5 Gewichtsprozent Natriumaluminiumsilikat
15 Gewichtsprozent Cetylpolyglykoläther mit 8 Mol Äthylenoxid
2 Gewichtsprozent Spindelöl
10 Gewichtsprozent Polyäthylenglykol
23 Teile Wasser

C. Emulsionkonzentrat

25 Gewichtsprozent Wirkstoff
15 Gewichtsprozent Cyclohexanon
55 Gewichtsprozent Xylol
5 Gewichtsprozent Mischung von Nonylphenylpolyoxyäthylen oder Calciumdodecylbenzolsulfonat.

Die neuen erfindungsgemäßen Verbindungen lassen sich zum Beispiel herstellen, indem man Verbindungen der allgemeinen Formel

-9-

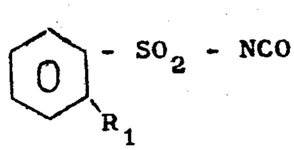

II

mit Verbindungen der allgemeinen Formel

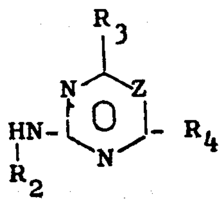

III

in Gegenwart eines inerten Lösungsmittels umsetzt, worin $R_1$, $R_2$, $R_3$, $R_4$ und Z die oben genannte Bedeutung haben.

Die Herstellung der erfindungsgemäßen Verbindungen der allgemeinen Formel I erfolgt durch Umsetzung eines Phenylsulfonyl-isocyanates der allgemeinen Formel II mit einem Amin der allgemeinen Formel III in einem inerten Lösungsmittel, wie Methylenchlorid, Acetonitril, Benzol, Toluol, 1,2-Dichloräthan, Tetrahydrofuran, Dimethyloxyäthan oder Diäthyläther, bei Temperaturen zwischen 0°C und dem Siedepunkt des jeweiligen Lösungsmittels, vorzugsweise bei 20°C bis 60°C.

Die Isocyanate der allgemeinen Formel II lassen sich aus entsprechenden Sulfamiden durch Umsetzung mit Oxalylchlorid in guten Ausbeuten herstellen.

Diese Sulfamide sind nach an sich bekannten Verfahren herstellbar (JOC 27, 1703 (1962); Khim. Farm. Zh. 1979, 36; CA 90, 203623 m).

Die Ausgangsverbindungen der allgemeinen Formel III sind an sich bekannt oder können nach an sich bekannten Verfahren hergestellt werden

-10-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

(D.J. Brown in "A. Weissberger, The Chemistry of Heterocyclic Compounds", Vol. XVI, "The Pyrimidines", John Wiley & Sons, New York a. London 1962

E.M. Smolin und L. Rapoport in "A. Weissberger, The Chemistry of Heterocyclic Compounds" Vol. XIII, "S-Triazines and Derivates", John Wiley & Sons, New York and London 1959)

Durch Kondensation eines Chlorheterocyclus mit einem ungesättigten Amin werden Alkenyl- und Alkinylaminoverbindungen der allgemeinen Formel III erhalten.

Dies geschieht vorzugsweise in einem inerten polaren Lösungsmittel, wie zum Beispiel Dimethylformamid, Nitroäthan, Tetrahydrofuran oder N-Methylpyrrolidon oder durch Umsetzung in dem entsprechenden Amin als Lösungsmittel. Die Verbindungen der Formel III sind stabile Substanzen, die sich durch Umkristallisieren oder Destillation reinigen lassen.

Die folgenden Beispiele erläutern die Herstellung der erfindungsgemäßen Verbindungen.

-11-

Schering Aktiengesellschaft
Gewerblicher Rechtsschutz



SCHERING

## BEISPIEL 1

**1-Allyl-3-(2-äthoxycarbonylphenylsulfonyl)-1-(4,6-dimethylpyrimidin-2-yl)-harnstoff**

Zu 1,95 g (12,00 mmol) 2-Allylamino-4,6-dimethylpyrimidin in 40 ml absolutem Toluol werden bei Raumtemperatur 3,31 g (13 mmol) 2-Äthoxycarbonylphenylsulfonylisocyanat getropft und 4 Stunden gerührt. Anschließend wird das Lösungsmittel abgezogen und der Rückstand an Kieselgel mit Essigester als Laufmittel chromatographiert.

Man erhält 3,5 g (70 % der Theorie) 1-Allyl-3-(2-äthoxycarbonylphenylsulfonyl)-1-(4,6-dimethylpyrimidin-2-yl)-harnstoff.
Fp.: 126-127°C

In analoger Weise lassen sich die folgenden erfindungsgemäßen Verbindungen herstellen.

-12-

| Beispiel Nr. | Name der Verbindungen | Physikalische Konstante |
|---|---|---|
| 2. | 1-Allyl-1-(4,6-dimethylpyrimidin-2-yl)-3-(2-methoxycarbonyl-phenylsulfonyl)-harnstoff | Fp.: 122-124°C |
| 3. | 1-Allyl-3-(2-methoxycarbonylphenylsulfonyl)-1-(4-methoxy-6-methylpyrimidin-2-yl)-harnstoff | Fp.: 91- 93°C |
| 4. | 1-Allyl-1-(4,6-dimethoxy-1,3,5-triazin-2-yl)-3-(2-methoxy-carbonylphenylsulfonyl)-harnstoff | Fp.: 128-130°C |
| 5. | 1-Allyl-1-(4-methoxy-6-methylpyrimidin-2-yl)-3-(2-äthoxy-carbonylphenylsulfonyl)-harnstoff | Fp.: 110-12°C |
| 6. | 1-Allyl-3-(2-äthoxycarbonylphenylsulfonyl)-1-(4-methoxy-6-methyl-1,3,5-triazin-2-yl)-harnstoff | Fp.: 70- 81°C |
| 7. | 1-Allyl-1-(4,6-dimethoxy-1,3,5-triazin-2-yl)-3-(2-äthoxy-carbonylphenylsulfonyl)-harnstoff | Fp.: 109-110°C |
| 8. | 1-Allyl-3-(2-propoxycarbonylphenylsulfonyl-1-pyrimidin-2-yl)-harnstoff | Fp.: 90°C |
| 9. | 1-Allyl-3-(2-propoxycarbonylphenylsulfonyl)-1-(4,6-dimethylpyrimidin-2-yl)-harnstoff | Fp.: 110°C |
| 10. | 1-Allyl-1-(4,6-dimethylpyrimidin-2-yl)-3-(2-isopropoxy-carbonylphenylsulfonyl)-harnstoff | Fp.: 154-155°C |
| 11. | 1-(4,6-Dimethylpyrimidin-2-yl)-3-(2-methoxycarbonylphenyl-sulfonyl)-1-propargylharnstoff | Fp.: 163°C |
| 12. | 1-(4,6-Dimethylpyrimidin-2-yl)-3-(2-äthoxycarbonylphenyl-sulfonyl)-1-propargyl-harnstoff | Fp.: 145-146°C |
| 13. | 1-Allyl-3-(2-chlorphenylsulfonyl)-1-(4,6-dimethylpyrimidin-2-yl)-harnstoff | Fp.: 127-131°C |
| 14. | 1-Allyl-3-(2-chlorphenylsulfonyl)-1-(4-methoxy-6-methyl-pyrimidin-2-yl)-harnstoff | Fp.: 119-123°C |

-13-

Schering Aktiengesellschaft
Gewerblicher Rechtsschutz





| Beispiel Nr. | Name der Verbindungen | Physikalische Konstante |
|---|---|---|
| 15. | 1-Allyl-3-(2-chlorphenylsulfonyl)-1-(4-methoxy-6-methyl-1,3,5-triazin-2-yl)-harnstoff | Fp.: 130-135°C |
| 16. | 1-Allyl-3-(2-chlorphenylsulfonyl)-1-(4,6-dimethoxy-1,3,5-triazin-2-yl)-harnstoff | Fp.: 149-152°C |
| 17. | 3-(2-Chlorphenylsulfonyl)-1-(4,6-dimethylpyrimidin-2-yl)-1-propargyl-harnstoff | Fp.: 158-160°C |
| 18. | 1-Allyl-1-(4-methoxy-6-methylpyrimidin-2-yl)-3-(2-propoxycarbonylphenylsulfonyl)-harnstoff | Fp.: 84- 91°C |
| 19. | 1-Allyl-1-(4,6-dimethoxy-1,3,5-triazin-2-yl)-3-(2-propoxycarbonylphenylsulfonyl)-harnstoff | Fp.: 118-120°C |
| 20. | 1-Allyl-3-/2-(2-chloräthoxy)-carbonylphenylsulfonyl7-1-(4,6-dimethylpyrimidin-2-yl)-harnstoff | Fp.: 141-142°C |
| 21. | 1-Allyl-3-/2-(2-chloräthoxycarbonyl)-phenylsulfonyl7-1-(4,6-dimethoxy-1,3,5-triazin-2-yl)-harnstoff | Fp.: 153-155°C |
| 22. | 1-Allyl-1-(4,6-dimethylpyrimidin-2-yl)-3-(2-N-morpholinocarbonylphenylsulfonyl)-harnstoff | Fp.: 153-154°C |
| 23. | 1-Allyl-1-(4,6-dimethylpyrimidin-2-yl)-3-(2-N-pyrolidinylcarbonylphenylsulfonyl)-harnstoff | Fp.: 163-165°C |
| 24. | 1-Allyl-3-(2-dimethylaminocarbonylphenylsulfonyl)-1-(4-methoxy-6-methylpyrimidin-2-yl)-harnstoff | Fp.: 157-159°C |

-14-

| Beispiel Nr. | Name der Verbindungen | Physikalische Konstante |
|---|---|---|
| 25. | 1-(2-Chlorallyl)-1-(4,6-dimethylpyrimidin-2-yl)-3-(2-methoxycarbonylphenylsulfonyl)-harnstoff | Fp.:149-152°C |
| 26. | 1-Allyl-3-(2-ethoxycarbonylphenylsulfonyl)-1-(pyrimidin-2-yl)-harnstoff | Fp.: 75- 77°C |
| 27. | 1-(2-Chlorallyl)-1-(4,6-dimethoxytriazin-2-yl)-3-methoxycarbonylphenylsulfonyl)-harnstoff | Fp.:115-120°C |
| 28. | 1-(1,1-Dichlorprop-1-en-3y)-1-(4,6-dimethylpyrimidin-2-yl)-3-(2-ethoxycarbonylphenylsulfonyl)-harnstoff | Fp.:138-140°C |
| 29. | 1-Crotyl-1-(4,6-dimethylpyrimidin-2-yl)-1-/2-(ethoxy-carbonyl)-phenylsulfonyl7-harnstoff | Fp.:141-142°C |
| 30. | 1-Crotyl-1-(4,6-dimethylpyridin-2-yl)-1-(2-(methoxycarbonyl)-phenylsulfonyl)-harnstoff | Fp.:110-12°C |
| 31. | 1-(4,6-Dimethylpyrimidin-2-yl)-1-propargyl-3-(2-propoxy-carbonylphenylsulfonyl)-harnstoff | Fp.:122-125°C |
| 32. | 1-(4,6-Dimethylpyrimidin-2-yl)-1-propargyl-3-(2-iso-propoxycarbonylphenylsulfonyl)-harnstoff | Fp.:132-133°C |
| 33. | 3-(2-Butoxycarbonylphenylsulfonyl)-1-(4,6-dimethyl-pyrimidin-2-yl)-1-propargyl-harnstoff | Fp.:125-136°C |
| 34. | 3-(2-Chlorphenylsulfonyl)-1-(3,3-dichlorprop-2-enyl)-1-(4,6-dimethylpyrimidin-2-yl)-harnstoff | Fp.:133-135°C |
| 35. | 1-(1,1-Dichlorprop-1-en-3-yl)-1-(4,6-dimethyl-pyrimidin-2-yl)-3-(2-methoxycarbonylphenylsulfonyl)-harnstoff | Fp.:140-142°C |
| 36. | 1-(2-Chlorallyl)-1-(4,6-dimethylpyrimidin-2-yl)-3-(2-ethoxycarbonylphenylsulfonyl)-harnstoff | Fp.:158-160°C |
| 37. | 1-Allyl-3-(2-n-butoxycarbonylsulfonyl)-1-(4-methoxy-6-methylpyrimidin-2-yl)-harnstoff | Fp.: 54- 55°C |
| 38. | 1-Allyl-3-(2-allyloxycarbonylphenylsulfonyl)-1-(4,6-dimethylpyrimidin-2-yl)-harnstoff | Fp.:108-110°C |
| 39. | 1-Allyl-3-(2-chlorphenylsulfonyl)-1-(4-chlor-6-methyl-1,3,5-triazin-2-yl)-harnstoff | Fp.:169-174°C |

-15-

| Beispiel Nr. | Name der Verbindungen | Physikalische Konstante |
|---|---|---|
| 55. | 1-(4,6-Dimethylpyrimidin-2-yl)-3-(2-ethoxycarbonylphenylsulfonyl)-1-methallylharnstoff | Fp.:194-195°C Zersetzung |
| 56. | 1-(2-Chlorallyl)-3-(2-chlorphenylsulfonyl)-1-(4,6-dimethyl-1,3,5-triazin-2-yl)-harnstoff | Fp.:202-203°C Zersetzung |
| 57. | 1-(3,3-Dichlorallyl)-1-(4,6-dimethyl-1,3,5-triazin-2-yl)-3-(2-methoxycarbonylphenylsulfonyl)-harnstoff | Fp.:180-182°C Zersetzung |
| 58. | 1-(3,3-Dichlorallyl)-1-(4,6-dimethyl-1,3,5-triazin-2-yl)-3-(2-ethoxycarbonylphenylsulfonyl)-harnstoff | Fp.:186-188°C Zersetzung |
| 59. | 1-(3-Chlorallyl)-1-(4,6-dimethoxy-1,3,5-triazin-2-yl)-3-(2-chlorbenzolsulfonyl)-harnstoff | Fp.:126-128°C |
| 60. | 1-(3-Chlorallyl)-1-(4,6-dimethylpyrimidin-2-yl)-3-(2-methoxycarbonylbenzolsulfonyl)-harnstoff | Fp.:133-137°C |
| 61. | 1-Allyl-1-(4-N,N-dimethylamino-6-methyl-1,3,5-triazin-2-yl)-3-(2-methoxycarbonylbenzolsulfonyl)-harnstoff | Fp.:144-146°C |
| 62. | 1-(4-ethoxy-6-methyl-1,3,5-triazin-2-yl)-1-allyl-3-(2-methoxycarbonylbenzolsulfonyl)-harnstoff | Fp.:103-105°C |
| 63. | 1-(3-Chlorallyl)-1-(4,6-dimethoxy-1,3,5-triazin-2-yl)-3-(2-methoxycarbonylbenzolsulfonyl)-harnstoff | Fp.: - |
| 64. | 1-Allyl-1-1-(4-(N,N-dimethylamino)-6-methoxy-1,3,5-triazin-2-yl)-3-(2-ethoxycarbonylbenzolsulfonyl)-harnstoff | Fp.:161-163°C (Zersetzung) |
| 65. | 1-Allyl-3-(2-ethoxycarbonylbenzolsulfonyl)-1-(4-methoxy-6-propinyloxy-1,3,5-triazin-2-yl)-harnstoff | |
| 66. | 1-Allyl-3-(2-chlorbenzolsulfonyl)-1-(4,6-dimethoxy-ethoxy-1,3,5-triazin-2-yl)-harnstoff | |
| 67. | 1-(3-Chlorallyl)-1-(4-methoxy-6-methyl-1,3,5-triazin-2-yl)-3-(2-methoxycarbonylbenzolsulfonyl)-harnstoff | Fp.:108-110°C |
| 68. | 1-(2-Chlorallyl)-1-(4,6-dimethyl-1,3,5-triazin-2-yl)-3-(2-methoxycarbonylphenylsulfonyl)-harnstoff | Fp.: 70-74°C |
| 69. | 1-(2-Chlorallyl)-1-(4,6-dimethyl-1,3,5-triazin-2-yl)-3-(2-ethoxycarbonylphenylsulfonyl)-harnstoff | Öl |

-16-

Postanschrift: Schering Aktiengesellschaft, Postfach 65 03 11, D-1000 Berlin 65 · Für Besucher Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

| Beispiel Nr. | Name der Verbindungen | Physikalische Konstante |
|---|---|---|
| 40 . | 1-Allyl-1-(4-(N,N-dimethylamino)-6-methoxy-1,3,5-triazin-2-yl)-3-(2-methoxycarbonylphenylsulfonyl)-harnstoff | Fp.:132,5-138°C |
| 41 . | 1-Allyl-3-(2-methoxycarbonylphenylsulfonyl)-1-(4-methoxy-6-propinyloxy-1,3,5-triazin-2-yl)-harnstoff | Öl |
| 42 . | 1-Allyl-3-(2-chlorphenylsulfonyl)-1-(4-ethoxy-6-methoxy-1,3,5-triazin-2-yl)-harnstoff | Fp.: 95-102°C |
| 43 . | 1-Allyl-3-(2-chlorphenylsulfonyl)-1-(4-methoxy-1,3,5-triazin-2-yl)-harnstoff | Fp.:104-111°C |
| 44 . | 1-(3-Chlorallyl)-3-(2-chlorphenylsulfonyl)-1-(4,6-dimethoxy-1,3,5-triazin-2-yl)-harnstoff | Fp.:128-136°C |
| 45 . | 3-(2-Chlorphenylsulfonyl)-2-(4-chlor-6-methyl-1,3,5-triazin-2-yl)-1-propargyl-harnstoff | Fp.:130-135°C |
| 46 . | 1-Allyl-3-(2-chlorphenylsulfonyl)-1-(4-(N,N-dimethylamino)-6-methoxy-1,3,5-triazin-2-yl)-harnstoff | |
| 47 . | 3-(2-Chlorphenylsulfonyl)-1-(3,3-dichlorprop-2-enyl)-1-4,6-dimethylpyrimidin-2-yl)-harnstoff | Fp.:133-135°C Zersetzung |
| 48 . | 1-(3,3-Dichlorprop-2-enyl)-1-(4,6-dimethyl-pyrimidin-2-yl)-3-(2-methoxycarbonylphenylsulfonyl)-harnstoff | Fp.: 140-142°C (Zersetzung) |
| 49 . | 1-(3,3-Dichlorprop-2-enyl)-1-(4,6-dimethylpyrimidin-2-yl)-3-(2-ethoxycarbonylphenylsulfonyl)-harnstoff | Fp.:138-140°C |
| 50 . | 1-(3,3-Dimethylallyl)-1-(4,6-dimethylpyrimidin-2-yl)-3-(2-ethoxycarbonylphenylsulfonyl)-harnstoff | Fp.:165-166°C Zersetzung |
| 51 . | 1-(4,6-Dimethylpyrimidin-2-yl)-3-(2-methoxy-carbonylphenylsulfonyl)-1-(3-phenylallyl)-harnstoff | Fp.:178-179°C Zersetzung |
| 52 . | 1-(4,6-Dimethylpyrimidin-2-yl)-3-(2-ethoxycarbonylphenylsulfonyl)-1-(3-phenylallyl)-harnstoff | Fp.:186-188°C |
| 53 . | 1-(4,6-Dimethylpyrimidin-2-yl)-1-methylallyl-3-(2-methoxycarbonylphenylsulfonyl)-harnstoff | Fp.:190-191°C Zersetzung |
| 54 . | 3-(2-Chlorphenylsulfonyl)-1-(2,4-dimethylpyrimidin-2-yl)-1-methallylharnstoff | Fp.:191-192°C Zersetzung |

-17-

| Beispiel Nr. | Name der Verbindungen | Physikalische Konstante |
|---|---|---|
| 70. | 3-(2-Chlorphenylsulfonyl)-1-(3,3-dichlorallyl)-1-(4,6-dimethyl-1,3,5-triazin-2-yl)-harnstoff | Fp.:174-176°C |
| 71. | 1-(2-Chlorbenzolsulfonyl)-3-(4-methoxy-6-methyl-1,3,5-triazin-2-yl)-3-propargyl-harnstoff | Fp.:144-147°C |
| 72. | 1-(2-Methoxycarbonylbenzolsulfonyl)-3-(4-methoxy-6-methyl-1,3,5-triazin-2-yl)-3-propargyl-harnstoff | Fp.:120-122°C |
| 73. | 1-(3-Chlorallyl)-1-(4,6-dimethoxy-1,3,5-triazin-2-yl)-3-(2-methoxycarbonylbenzolsulfonyl)-harnstoff | harziges Öl |

Nach dem im Beispiel 1 beschriebenen Verfahren lassen sich weiterhin auch erfindungsgemäße Verbindungen herstellen, die durch die in der folgenden Tabelle aufgeführten Substituenten gekennzeichnet sind.

-18-

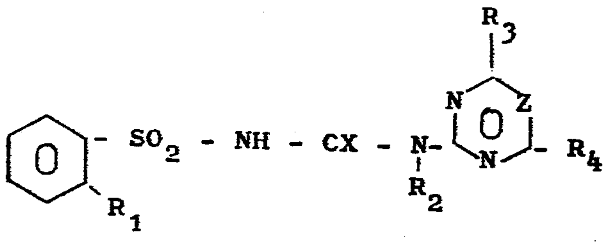

mit

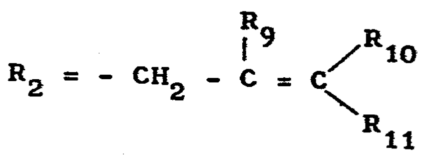 und $-CH_2-C\equiv C-R_{11}$

| Beispiel Nr. | $R_1$ | $R_9$ | $R_{10}$ | $R_{11}$ | $R_3$ | $R_4$ | Z | X |
|---|---|---|---|---|---|---|---|---|
| 46 | $CO_2CH_3$ | H | H | H | $CH_3$ | H | CH | O |
| 47 | $CO_2CH_3$ | H | H | H | $OCH_3$ | $OCH_3$ | CH | O |
| 48 | $CO_2CH_3$ | H | H | H | H | H | CH | O |
| 49 | $CO_2CH_3$ | H | H | H | $CH_3$ | OEt | CH | O |
| 50 | $CO_2CH_3$ | H | H | H | OEt | OEt | CH | O |
| 51 | $CO_2CH_3$ | H | H | H | $CH_3$ | $CH_3$ | N | O |
| 52 | $CO_2CH_3$ | H | H | H | $CH_3$ | $OCH_3$ | N | O |
| 53 | $CO_2CH_3$ | H | H | H | $OCH_3$ | OEt | N | O |
| 54 | $CO_2CH_3$ | H | H | H | $CH_3$ | OEt | N | O |
| 55 | $CO_2CH_3$ | H | H | H | $OCH_3$ | Et | N | O |
| 56 | $CO_2CH_3$ | H | H | H | $CH_3$ | Et | N | O |
| 57 | $CO_2CH_3$ | H | H | H | $NMe_2$ | $CH_3$ | N | O |
| 58 | $CO_2CH_3$ | H | H | H | $NEt_2$ | $CH_3$ | N | O |

-19-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: ... Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. ... · Berliner Handels- und Frankfurter Bank, Berlin, Konto-Nr. 70045224, Bankleitzahl 100 202 00 · Deutsche Bank Berlin AG, Konto-Nr. 2415008 ... · Postscheckamt Berlin West, Konto-Nr. 1175-101, Bankleitzahl 100 100 10

| Beispiel Nr. | $R_1$ | $R_9$ | $R_{10}$ | $R_{11}$ | $R_3$ | $R_4$ | Z | X |
|---|---|---|---|---|---|---|---|---|
| 59 | $CO_2iPr$ | H | H | H | H | $CH_3$ | CH | O |
| 60 | $CO_2iPr$ | H | H | H | $CH_3$ | $OCH_3$ | CH | O |
| 61 | $CO_2iPr$ | H | H | H | $OCH_3$ | $OCH_3$ | N | O |
| 62 | $CO_2iPr$ | H | H | H | H | H | CH | O |
| 63 | $CO_2CH_2CH_2OCH_3$ | H | H | H | H | H | CH | O |
| 64 | $CO_2CH_2CH_2OCH_3$ | H | H | H | $CH_3$ | $CH_3$ | CH | O |
| 65 | $CO_2CH_2CH_2OCH_3$ | H | H | H | $CH_3$ | $OCH_3$ | N | O |
| 66 | $CO_2CH_2CH_2OCH_3$ | H | H | H | $OCH_3$ | $OCH_3$ | N | O |
| 67 | $CO_2CH_3$ | Cl | H | H | $CH_3$ | $OCH_3$ | CH | O |
| 68 | $CO_2Et$ | Cl | H | H | $CH_3$ | $CH_3$ | CH | O |
| 69 | $CO_2Et$ | H | Cl | $CH_3$ | $CH_3$ | $CH_3$ | CH | O |
| 70 | $CO_2CH_3$ | H | $C_6H_5$ | H | $CH_3$ | $CH_3$ | CH | O |
| 71 | $CO_2Et$ | H | $C_6H_5$ | H | $CH_3$ | $CH_3$ | CH | O |
| 72 | $CO_2Pr$ | H | $C_6H_5$ | H | $CH_3$ | $CH_3$ | CH | O |
| 73 | $CO_2CH_3$ | H | H | CN | $CH_3$ | $CH_3$ | CH | O |
| 74 | $CO_2Et$ | H | H | CN | $CH_3$ | $CH_3$ | CH | O |
| 75 | $CO_2CH_3$ | H | $CH_3$ | $CH_3$ | $CH_3$ | $CH_3$ | CH | O |
| 76 | $CO_2Et$ | H | $CH_3$ | $CH_3$ | $CH_3$ | $CH_3$ | CH | O |
| 77 | $CO_2Pr$ | H | $CH_3$ | $CH_3$ | $CH_3$ | $CH_3$ | CH | O |
| 78 | $CO_2Et$ | - | - | H | $OCH_3$ | $CH_3$ | CH | O |
| 79 | $CO_2Et$ | - | - | H | $OCH_3$ | $OCH_3$ | N | O |
| 80 | $CONMe_2$ | H | H | H | $CH_3$ | $CH_3$ | CH | O |

-20-

**Postanschrift: Schering Aktiengesellschaft, Postfach 65 03 11, D-1000 Berlin 65** · Für Besucher: Berlin-Wedding, Müllerstraße 170-178 · Telegramme: Scheringchemie Berlin.

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

| Beispiel Nr. | $R_1$ | $R_9$ | $R_{10}$ | $R_{11}$ | $R_3$ | $R_4$ | Z | X |
|---|---|---|---|---|---|---|---|---|
| 81 | Cl | - | - | $CH_3$ | $CH_3$ | $CH_3$ | CH | O |
| 82 | $SO_2CH_3$ | H | H | H | $CH_3$ | $CH_3$ | CH | O |
| 83 | $SO_2CH_3$ | H | H | H | $OCH_3$ | $OCH_3$ | N | O |
| 84 | $SO_2CH_3$ | - | - | H | $CH_3$ | $CH_3$ | CH | O |
| 85 | $SO_2C_4H_9$ | H | H | H | $CH_3$ | $CH_3$ | CH | O |
| 86 | $CO_2Et$ | H | H | $CF_3$ | $CH_3$ | $CH_3$ | CH | O |

-21-

Die Ausgangsverbindungen zur Herstellung der erfindungsgemäßen Verbindungen sind, wie oben erläutert wurde, bereits an sich bekannt oder können nach an sich bekannten Verfahren hergestellt werden.

Die folgenden Beispiele dienen zur Erläuterung der Herstellung der Ausgangsverbindungen.

BEISPIEL 76

2-Äthoxycarbonylphenylsulfonylisocyanat

Zu 57,1 g (0,45 Mol) Oxalylchlorid und 0,4 g Diazabicyclo /2.2.2/ octan in 500 ml absolutem Toluol werden bei 85°C 68,76 g (0,3 Mol) 2-Sulfamylbenzoesäureäthylester gegeben. Das Reaktionsgemisch wird noch 3 Stunden auf eine Innentemperatur von 95°C erhitzt bis keine Gasentwicklung mehr beobachtet wird.

Das überschüssige Oxalylchlorid und Toluol wird weitgehend abdestilliert, vom Feststoff dekantiert und das Produkt im Hochvakuum destilliert. Man erhält 63,5 g (83 % der Theorie) hellgelbes Öl.

Kp.: 148°C/0,5 Torr



Postanschrift: Schering Aktiengesellschaft, Postfach 65 03 11, D-1000 Berlin 65 · Fur Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

BEISPIEL 77

2-Sulfamylbenzoesäuremethylester

In eine Suspension von 143,3 g (0,78 Mol) in 600 ml absolutem Methanol wird unter Kühlung ein H Cl-Strom bis zur Sättigung geleitet. Anschließend wird die Lösung noch 2 Stunden refluxiert bis das Sacharin vollständig in Lösung gegangen und die Umsetzung beendet ist.

Der überschüssige Alkohol wird abgedampft, der Rückstand in Essigester aufgenommen und mit wenig gesättigter $Na_2$-$CO_3$-Lösung zur Abtrennung nicht umgesetzten Sacharins gewaschen. Man erhält nach Abdampfen des Essigesters im Vakuum 148,1 g (88 % der Theorie) 2-Sulfamylbenzoesäureäthylester vom Fp.: 122-124°C

Analog werden folgende 2-Sulfamylbenzoesäureester hergestellt:
Äthyl-, n-Propyl-, iso-Propyl-, 2-Methoxyäthyl-, 2-Äthoxyäthyl-, Allyl-, Butyl-, Hexyl-, Cyclopentyl-, 2-Chloräthyl.

-23-

BEISPIEL 78

2-Allylamino-4,6-dimethylpyrimidin

20 g (0,14 Mol) 2-Chlor-4,6-dimethylpyrimidin werden in 60 ml Allylamin unter Rückfluß erhitzt. Nach 4 Stunden ist die Reaktion beendet. Das überschüssige Allylamin wird abdestilliert, der Rückstand in Essigester aufgenommen und mit 150 ml gesättigter Bicarbonatlösung gewaschen. Nach Trocknen über $Na_2SO_4$ wird das Lösungsmittel abgezogen. Man erhält 22,5 g (99 % der Theorie) 2-Allylamino-4,6-dimethylpyrimidin.
Fp.: 68-70°C

Analog lassen sich die folgenden Alkenylaminopyrimidine und -triazine aus entsprechenden 2-Chlorheterocyclen synthetisieren:

2-(2-Chlorallylamino)-4,6-dimethylpyrimidin
2-Crotylamino-4,6-dimethylpyrimidin
2-Prenylamino-4,6-dimethylpyrimidin
2-(1,1,2-Trichlorprop-1-en-3-ylamino)-4,6-dimethylpyrimidin
2-Allylaminopyrimidin
2-Allylamino-4-methoxy-6-methylpyrimidin
2-Propargylamino-4,6-dimethylpyrimidin
2-Propargylamino-4-methoxy-6-methylpyrimidin
2-(But-2-in-4-ylamino)-4,6-dimethylpyrimidin
2-(4,4,4-Trifluorcrotylamino)-4,6-dimethylpyrimidin
2-Methallylamino-4,6-dimethylpyrimidin
2-(3-Phenylallylamino)-4,6-dimethylpyrimidin
2-Allylamino-4,6-dimethyl-1,3,5-triazin
2-Allylamino-4-methoxy-6-methyl-1,3,5-triazin
2-Allylamino-4,6-dimethoxy-1,3,3-triazin
2-Allylamino-4,6-diäthoxy-1,3.5-triazin
2-Allylamino-4,6-dimethoxypyrimidin



Postanschrift: Schering Aktiengesellschaft, Postfach 65 03 11, D-1000 Berlin 65 · Fur Besucher Berlin-Wedding, Mullerstraße 170-178 · Telegramme Scheringchemie Berl
Vorstand Dr Herbert Asmis Dr Christian Bruhn Dr Heinz Hannse Horst Kramp, Dr Klaus Pohle, Dr. Horst Witzel · Vorsitzender des Aufsichtsrats: Hans-Jürgen Hamar
Sitz der Gesellschaft Berlin und Bergkamen · Handelsregister AG Charlottenburg 93 HRB 283 und AG Kamen HRB 0061 · Berliner Commerzbank AG, Berlin, Konto-N
108700600 Bankleitzahl 100 400 00 · Berliner Handels- und Frankfurter Bank, Berlin, Konto-Nr 70045224, Bankleitzahl 100 202 00 · Deutsche Bank Berlin AG, Konto-N
2415008, Bankleitzahl 100 700 00 · Postscheckamt Berlin West Konto-Nr 1175-101, Bankleitzahl 100 100 10

Die folgenden Beispiele dienen zur Erläuterung der Anwendungsmöglichkeiten der erfindungsgemäßen Verbindungen, die in Form der oben angeführten Zubereitungen erfolgten:

BEISPIEL 79

Im Gewächshaus wurden die in der Tabelle aufgeführten Verbindungen in einer Aufwandmenge von 3,0 kg Wirkstoff/ha suspendiert in 500 Liter Wasser/ha auf Helianthus und Chrysanthemum als Testpflanzen im Vor- und Nachauflaufverfahren gespritzt.

Die Schädigung der Unkräuter wurde in einem Boniturschema von 0 bis 4 3 Wochen nach der Behandlung bonitiert, wobei bedeutet:

0 = keine Wirkung
1 = mittlere Wuchshemmung
2 = starke Wuchshemmung
3 = völlige Wuchshemmung
4 = total vernichtet

VA = Vorauflauf
NA = Nachauflauf



Postanschrift: Schering Aktiengesellschaft, Postfach 65 03 11, D-1000 Berlin 65 · Fur Besucher: Berlin-Wedding, Mullerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann · Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

| Erfindungsgemäße Verbindungen | Helianthus VA | Helianthus NA | Chrysanthemum VA | Chrysanthemum NA |
|---|---|---|---|---|
| 1-Allyl-1-(4,6-dimethylpyrimidin-2-yl)-3-(2-methoxycarbonylphenylsulfonyl)-harnstoff | 4 | 4 | 4 | 4 |
| 1-Allyl-3-(2-methoxycarbonylphenylsulfonyl)-1-(4-methoxy-6-methylpyrimidin-2-yl)-harnstoff | 4 | 4 | 4 | 4 |
| 1-Allyl-1-(4,6-dimethoxy-1,3,5-triazin-2-yl)-3-(2-methoxycarbonylphenylsulfonyl)-harnstoff | 4 | 4 | 4 | 4 |
| 1-Allyl-1-(4-methoxy-6-methylpyrimidin-2-yl)-3-(2-äthoxycarbonylphenylsulfonyl)-harnstoff | 4 | 4 | 4 | 4 |
| 1-Allyl-3-(2-äthoxycarbonylphenylsulfonyl)-1-(4-methoxy-6-methyl-1,3,5-triazin-2-yl)-harnstoff | 4 | 4 | 4 | 4 |
| 1-Allyl-1-(4,6-dimethoxy-1,3,5-triazin-2-yl)-3-(2-äthoxycarbonylphenylsulfonyl)-harnstoff | 4 | 4 | 4 | 4 |
| 1-Allyl-3-(2-propoxycarbonylphenylsulfonyl-1-(pyrimidin-2-yl)-harnstoff | 4 | 4 | 4 | 4 |
| 1-Allyl-3-(2-propoxycarbonylphenylsulfonyl)-1-(4,6-dimethylpyrimidin-2-yl)-harnstoff | 4 | 4 | 4 | 4 |
| 1-Allyl-1-(4,6-dimethylpyrimidin-2-yl)-3-(2-methoxycarbonylphenylsulfonyl)-1-propargylharnstoff | 4 | 4 | 4 | 4 |
| 1-(4,6-Dimethylpyrimidin-2-yl)-3-(2-äthoxycarbonylphenylsulfonyl)-1-propargylharnstoff | 4 | 4 | 4 | 4 |
| 1-Allyl-3-(2-chlorphenylsulfonyl)-1-(4,6-dimethylpyrimidin-2-yl)-harnstoff | 4 | 4 | 4 | 4 |
| 1-Allyl-3-(2-chlorphenylsulfonyl)-1-(4-methoxy-6-methyl-pyrimidin-2-yl)-harnstoff | 4 | 4 | 4 | 4 |
| 1-Allyl-3-(2-chlorphenylsulfonyl)-1-(4,6-dimethoxy-1,3,5-triazin-2-yl)-harnstoff | 4 | 4 | 4 | 4 |
| 3-(2-Chlorphenylsulfonyl)-1-(4,6-dimethylpyrimidin-2-yl)-1-propargyl-harnstoff | 4 | 4 | 4 | 4 |



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann · Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

| Erfindungsgemäße Verbindungen | Helianthus | | Chrysanthemum | |
|---|---|---|---|---|
| | VA | NA | VA | NA |
| 1-Allyl-3-(2-äthoxycarbonylphenylsulfonyl)-1-(4-methoxy-6-methyl-1,3,5-triazin-2-yl)-harnstoff | 4 | 4 | 4 | 4 |
| 1-Allyl-1-(4,6-dimethylpyrimidin-2-yl)-3-(2-isopropoxycarbonylphenylsulfonyl)-harnstoff | 4 | 4 | 4 | 4 |
| 1-Allyl-3-(2-chlorphenylsulfonyl)-1-(4-methoxy-6-methyl-1,3,5-triazin-2-yl)-harnstoff | 4 | 4 | 4 | 4 |
| 1-Allyl-1-(4-methoxy-6-methylpyrimidin-2-yl)-3-(propoxycarbonylphenylsulfonyl)-harnstoff | 4 | 4 | 4 | 4 |
| 1-Allyl-1-(4,6-dimethoxy-1,3,5-triazin-2-yl)-3-(2-propoxycarbonylphenylsulfonyl)-harnstoff | 4 | 4 | 4 | 4 |
| 1-Allyl-1-(4,6-dimethylpyrimidin-2-yl)-3-(2-N-pyrolidinylcarbonylphenylsulfonyl)-harnstoff | 4 | 4 | 4 | 4 |
| 1-Allyl-3-(2-dimethylaminocarbonylphenylsulfonyl)-1-(4-methoxy-6-methylpyrimidin-2-yl)-harnstoff | 4 | 4 | 4 | 4 |
| 1-Allyl-3-/2-(2-chloräthoxy)-carbonylphenylsulfonyl/-1-(4,6-dimethylpyrimidin-2-yl)-harnstoff | 4 | 4 | 4 | 4 |
| 1-Allyl-3-/2-(2-chloräthoxycarbonyl)-phenylsulfonyl/-1-(4,6-dimethoxy-1,3,5-triazin-2-yl)-harnstoff | 4 | 4 | 4 | 4 |
| 1-(2-Chlorallyl)-1-(4,6-dimethylpyrimidin-2-yl)-3-(2-methoxycarbonylphenylsulfonyl)-harnstoff | 4 | 4 | 4 | 4 |
| 1-Allyl-3-(2-ethoxycarbonylphenylsulfonyl)-1-(pyrimidin-2-yl)-harnstoff | 4 | 4 | 4 | 4 |
| 1-(2-Chlorallyl)-1-(4,6-dimethoxytriazin-2-yl)-3-(methoxycarbonylphenylsulfonyl)-harnstoff | 4 | 4 | 4 | 4 |
| 1(1,1-Dichlorprop-1-en-3y)-1-(4,6 dimethylpyrimidin-2-yl)-3-(2-ethoxycarbonylphenylsulfonyl)-harnstoff | 4 | 4 | 4 | 4 |

-27-

Postanschrift: Schering Aktiengesellschaft, Postfach 65 03 11, D-1000 Berlin 65 · Fur Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

| Erfindungsgemäße Verbindungen | Helianthus VA | Helianthus NA | Chrysanthemum VA | Chrysanthemum NA |
|---|---|---|---|---|
| 1-Crotyl-1-(4,6-dimethyl-pyrimidin-2-yl)-1-[2-(ethoxy-carbonyl)phenylsulfonyl]-harnstoff | 4 | 4 | 4 | 4 |
| 1-Crotyl-1-(4,6-dimethyl-pyrimidin-2-yl)-1-[2-(methoxy-carbonyl)phenylsulfonyl]-harnstoff | 4 | 4 | 4 | 4 |
| 1-(4,6-Dimethylpyrimidin-2-yl)-1-propargyl-3-(2-propoxycarbonyl-phenylsulfonyl)-harnstoff | 4 | 4 | 4 | 4 |
| 1-(4,6-Dimethylpyrimidin-2-yl)-1-propargyl-3-(2-isopropoxycarbonylphenylsulfonyl)-harnstoff | 4 | 4 | 4 | 4 |
| 3-(2-Chlorphenylsulfonyl)-1-(3,3-dichlorprop-2-enyl)-1-(4,6-dimethylpyrimidin-2-yl)-harnstoff | 4 | 4 | 4 | 4 |
| 1-(1,1-Dichlorprop-1-en-3-yl)-1-(4,6-dimethyl-pyrimidin-2-yl)-3-(2-methoxycarbonylphenyl-sulfonyl)-harnstoff | 4 | 4 | 4 | 4 |
| 1-(2-Chlorallyl)-1-(4,6-dimethyl-pyrimidin-2-yl)-3-(2-ethoxycarbonylphenylsuflonyl)-harnstoff | 4 | 4 | 4 | 4 |
| 1-Allyl-3-(2-allyloxycarbonyl-phenylsulfonyl)-1-1(4,6-dimethyl-pyrimidin-2-yl)-harnstoff | 4 | 4 | 4 | 4 |
| 1-Allyl-3-(2-chlorphenylsulfonyl)-1-(4-chlor-6-methyl-1,3,5-triazin-2-yl)-harnstoff | 4 | 4 | 4 | 4 |
| 1-Allyl-1-[4-(N,N-dimethylamino)-6-methoxy-1,3,5-triazin-2-yl]-3-(2-methoxycarbonylphenylsulfonyl-harnstoff | 4 | 4 | 4 | 4 |
| 1-Allyl-3-(2-methoxycarbonyl-phenylsulfonyl)-1-(4-methoxy-6-propinyloxy-1,3,5-triazin-2-yl)-harnstoff | 4 | 4 | 4 | 4 |

-28-

Schering Aktiengesellschaft
Gewerblicher Rechtsschutz



SCHERING

| Erfindungsgemäße Verbindungen | Helianthus VA | Helianthus NA | Chrysanthemum VA | Chrysanthemum NA |
|---|---|---|---|---|
| 1-Allyl-3-(2-chlorphenylsulfonyl)-1-(4-ethoxy-6-methoxy-1,3,5-triazin-2-yl)-harnstoff | 4 | 4 | 4 | 4 |
| 1-Allyl-3-(2-chlorphenylsulfonyl)-1-(4-methoxy-1,3,5-triazin-2-yl)-harnstoff | 4 | 4 | 4 | 4 |
| 1-(3-Chlorallyl)-3-(2-chlorphenylsulfonyl)-1-(4,6-dimethoxy-1,3,5-triazin-2-yl)-harnstoff | 4 | 4 | 4 | 4 |
| 3-(2-Chlorphenylsulfonyl)-2-(4-chlor-6-methyl-1,3,5-triazin-2-yl)-1-propargyl-harnstoff | 4 | 4 | 4 | 4 |
| 1-Allyl-3-(2-chlorphenylsulfonyl)-1-[4-(N,N-dimethylamino)-6-methoxy-1,3,5-triazin-2-yl]-harnstoff | 4 | 4 | 4 | 4 |
| 1-(3,3-Dimethylallyl)-1-4,6-dimethyl-pyrimidin-2-yl)-3-(2-ethoxycarbonylphenylsulfonyl)-harnstoff | 4 | 4 | 4 | 4 |
| 1-(4,6-Dimethylpyrimidin-2-yl)-3-(2-methoxy-carbonylphenylsulfonyl)-1-(3-phenylallyl)-harnstoff | 4 | 4 | 4 | 4 |
| 1-(4,6-Dimethylpyrimidin-2-yl)-3-(2-ethoxycarbonylphenylsulfonyl)-1-(3-phenylallyl)-harnstoff | 4 | 4 | 4 | 4 |
| 1-(4,6-Dimethylpyrimidin-2-yl)-1-methallyl-3-(2-methoxycarbonylphenylsulfonyl)-harnstoff | 4 | 4 | 4 | 4 |
| 3-(2-Chlorphenylsulfonyl)-1-(2,4-dimethylpyrimidin-2-yl)1-methallylharnstoff | 4 | 4 | 4 | 4 |



**Postanschrift. Schering Aktiengesellschaft, Postfach 65 03 11, D-1000 Berlin 65** · Fur Besucher: Berlin-Wedding, Müllerstraße 170-178 · Telegramme: Scheringchemie Berlin.

Vorstand: Dr Herbert Asmis Dr Christian Bruhn, Dr Heinz Hannse, Horst Kramp, Dr. Klaus Pohle, Dr. Horst Witzel · Vorsitzender des Aufsichtsrats: Hans-Jürgen Hamann
Sitz der Gesellschaft Berlin und Bergkamen Handelsregister AG Charlottenburg 93 HRB 283 und AG Kamen HRB 0061 · Berliner Commerzbank AG, Berlin, Konto-Nr. 108700600 Bankleitzahl 100 400 00 Berliner Handels- und Frankfurter Bank, Berlin, Konto-Nr. 70045224, Bankleitzahl 100 202 00 · Deutsche Bank Berlin AG, Konto-Nr. 2415008 Bankleitzahl 100 700 00 Postscheckamt Berlin West, Konto-Nr 1175-101, Bankleitzahl 100 100 10

Schering Aktiengesellschaft
Gewerblicher Rechtsschutz



SCHERING

| Erfindungsgemäße Verbindungen | Helianthus VA | Helianthus NA | Chrysanthemum VA | Chrysanthemum NA |
|---|---|---|---|---|
| 1-(4,6-Dimethylpyrimidin-2-yl)-3-(2-ethoxycarbonyl-phenylsulfonyl)-1-methallyl-harnstoff | 4 | 4 | 4 | 4 |
| 1-(2-Chlorallyl)-3-(2-chlorphenyl-sulfonyl)-1-(4,6-dimethyl-1,3,5-triazin-2-yl)-harnstoff | 4 | 4 | 4 | 4 |
| 1-(3,3-Dichlorallyl)-1-(4,6-dimethyl-1,3,5-triazin-2-yl)-3-(2-methoxycarbonylphenylsulfonyl)-harnstoff | 4 | 4 | 4 | 4 |
| 1-(3,3-Dichlorallyl)-1-(4,6-dimethyl-1,3,5-triazin-2-yl)-3-(2-ethoxycarbonylphenylsulfonyl)-harnstoff | 4 | 4 | 4 | 4 |
| 1-(3-Chlorallyl)-1-(4,6-dimethoxy-1,3,5-triazin-2-yl)-3-(2-chlorbenzol-sulfonyl)-harnstoff | 4 | 4 | 4 | 4 |
| 1-(3-Chlorallyl)-1-(4,6-dimethyl-pyrimidin-2-yl)-3-(2-methoxycarbonylbenzolsulfonyl)-harnstoff | 4 | 4 | 4 | 4 |
| 1-Allyl-1-(4-N,N-dimethylamino-6-methyl-1,3,5-triazin-2-yl)-3-(2-methoxycarbonylbenzolsulfonyl)-harnstoff | 4 | 4 | 4 | 4 |
| 1-(4-Äthoxy-6-methyl-1,3,5-triazin-2-yl)-1-allyl-3-(2-methoxycarbonyl-benzolsulfonyl)-harnstoff | 4 | 4 | 4 | 4 |
| 1-(3-Chlorallyl)-1-(4,6-dimethoxy-1,3,5-triazin-2-yl)-3-(2-methoxy-carbonyl-benzolsulfonyl)-harnstoff | 4 | 4 | 4 | 4 |
| 1-(2-Chlorbenzolsulfonyl)-3-(4-methoxy-6-methyl-1,3,5-triazin-2-yl)-3-propargyl-harnstoff | 4 | 4 | 4 | 4 |
| 1-(2-Methoxycarbonylbenzol-sulfonyl)-3-(4-methoxy-6-methyl-1,3,5-triazin-2-yl)-3-propargyl-harnstoff | 4 | 4 | 4 | 4 |
| 1-(3-Chlorallyl)-1-(4,6-dimethoxy-1,3,5-triazin-2-yl)-3-(2-methoxycarbonylbenzol-sulfonyl)-harnstoff | 4 | 4 | 4 | 4 |

-30-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

BEISPIEL 80

Samen mono- und dikotyler Unkräuter sowie die Kulturpflanzen Weizen und Soja wurden in Töpfe mit humushaltigem Sandboden ausgelegt und mit Erde abgedeckt. Die aufgeführten erfindungsgemäßen Verbindungen wurden als Suspension mit 500 Liter Wasser/ha in einer Aufwandmenge von 0,1 kg Wirkstoff/ha vor dem Auflaufen der Unkräuter auf die Erdoberfläche appliziert.

Nach der Behandlung wurden die Versuchstöpfe im Gewächshaus aufgestellt und die Versuchspflanzen unter guten Wuchsbedingungen kultiviert. 4 Wochen nach der Behandlung wurden die Pflanzenschäden bonitiert, wobei 0 = keine Wirkung und 4 = Vernichtung der Pflanzen bedeuten. Als Vergleich dienten dabei unbehandelte Kontrollen.

Wie aus der Tabelle ersichtlich wird, wurden alle Unkräuter vernichtet ohne Schädigung der Kulturpflanzen.

-31-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

Schering Aktiengesellschaft
Gewerblicher Rechtsschutz



SCHERING

| Erfindungsgemäße Verbindungen | Weizen | Soja | Helianthus | Stellaria | Abutilon | Viola | Amaranthus | Sesbania | Datura | Avena fatua | Alopecurus m. | Cyperus esculentus | Sorghum halenpense | Poa annua | Bromus tectorum |
|---|---|---|---|---|---|---|---|---|---|---|---|---|---|---|---|
| 1-(4,6-Dimethylpyrimidin-2-yl)-3-(2-äthoxycarbonylphenylsulfonyl)-1-propargyl-harnstoff | 0 | 0 | 3 | 3 | 3 | 3 | 3 | 3 | 3 | 2 | 2 | 2 | 2 | 2 | 2 |
| 1-Allyl-1-(4-methoxy-6-methylpyrimidin-2-yl)-3-(2-äthoxycarbonylphenylsulfonyl)-harnstoff | 0 | 0 | 4 | 4 | 3 | 4 | 4 | 4 | 4 | 2 | 3 | 2 | 3 | 3 | 3 |
| UNBEHANDELT | 0 | 0 | 0 | 0 | 0 | 0 | 0 | 0 | 0 | 0 | 0 | 0 | 0 | 0 | 0 |

-32-

Schering Aktiengesellschaft
Gewerblicher Rechtsschutz



SCHERING

BEISPIEL 81

Samen von zweikeimblättrigen Unkräutern und von Weizen und Soja wurden in Töpfen ausgesät und im Gewächshaus unter guten Wuchsbedingungen angezogen. 3 Wochen nach der Aussaat wurden die Versuchspflanzen im 1 bis 4-Blattstadium behandelt. Die Verbindungen wurden zu diesem Zweck als Suspensionen mit 500 Liter Wasser/ha in der unten angegebenen Aufwandmenge behandelt. 4 Wochen nach der Behandlung wurden die Pflanzenschäden nach dem Schema 0 = keine Wirkung und 4 = Vernichtung der Pflanzen bonitiert.
Auch hier zeigten die erfindungsgemäßen Verbindungen eine hohe Selektivität bei ausgezeichneter Wirkung gegen das Unkraut.

-33-

Schering Aktiengesellschaft
Gewerblicher Rechtsschutz



SCHERING

| Erfindungsgemäße Verbindungen | Aufwandmenge Wirkstoff/ha | Weizen | Soja | Helianthus | Brassica | Stellaria | Abutilon | Sida | Viola | Amaranthus | Sesbania | Datura | Euphorbia | Avena fatua | Alopecurus m. | Cyperus esculentus | Sorghum halepense | Poa annua | Bromus tectorum |
|---|---|---|---|---|---|---|---|---|---|---|---|---|---|---|---|---|---|---|---|
| 1-(4,6-Dimethylpyrimidin-2-yl)-3-(2-äthoxycarbonylphenylsulfon-yl)-1-propargyl-harnstoff | 0,1 | - | 0 | 3 | 3 | 3 | 3 | 3 | 3 | 3 | 3 | 3 | 3 | 2 | 2 | 2 | 2 | 2 | 2 |
| 1-Allyl-1-(4-methoxy-6-methyl-pyrimidin-2-yl)-3-(2-äthoxy-carbonylphenylsulfonyl)-harnstoff | 0,1 | - | 0 | 3 | 3 | 3 | 3 | 3 | 3 | 3 | 3 | 3 | 3 | 2 | 2 | 2 | 2 | 2 | 2 |
| 1-Allyl-1-(4,6-dimethoxy-1,3,5-triazin-2-yl)-3-(2-äthoxycarbonylphenylsulfonyl)-harnstoff | 0,03 | 0 | 0 | 3 | 3 | 3 | 3 | 3 | 3 | 3 | 3 | 3 | 3 | 3 | 3 | 3 | 3 | 3 | 3 |
| 1-Allyl-3-(2-äthoxycarbonyl-phenylsulfonyl)-1-(4-methoxy-6-methyl-1,3,5-triazin-2-yl)-harnstoff | 0,03 | 0 | 0 | 3 | 3 | 3 | 3 | 3 | 3 | 3 | 3 | 3 | 3 | 3 | 2 | 2 | 3 | 3 | 3 |



Postanschrift: Schering Aktiengesellschaft, Postfach 65 03 11, D-1000 Berlin 65 · Fur Besucher: Berlin-Wedding, Müllerstraße 170-178 · Telegramme: Scheringchemie Berlin

Vorstand: Dr. Herbert Asmis, Dr. Christian Brunn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

Schering Aktiengesellschaft
Gewerblicher Rechtsschutz





| Erfindungsgemäße Verbindungen | Aufwandmenge Wirkstoff/ha | Weizen | Soja | Helianthus | Brassica | Stellaria | Abutilon | Sida | Viola | Amaranthus | Sesbania | Datura | Euphorbia | Avena fatua | Alopecurus m. | Cyperus esculentus | Sorghum halepense | Poa annua | Bromus tectorum |
|---|---|---|---|---|---|---|---|---|---|---|---|---|---|---|---|---|---|---|---|
| 1-Allyl-1-(4,6-dimethyl-pyrimidin-2-yl)-3-(2-methoxy-carbonylphenylsulfonyl)-harnstoff | 0,03 | - | 0 | 3 | 3 | 3 | 3 | 3 | 3 | 3 | 3 | 3 | 3 | 3 | 3 | 3 | 3 | 3 | 3 |
| 1-Allyl-3-(2-chlorphenylsulfonyl)-1-(4,6-dimethoxy-1,3,5-triazin-2-yl)-harnstoff | 0,03 | 0 | 0 | 3 | 3 | 3 | 3 | 3 | 3 | 3 | 3 | 3 | 3 | - | - | - | - | - | - |
| 1-Allyl-3-(2-äthoxycarbonylphenylsulfonyl)-1-(4-methoxy-6-methyl-1,3,5-triazin-2-yl)-harnstoff | 0,03 | 0 | 0 | 3 | 3 | 3 | 3 | 3 | 3 | 3 | 3 | 3 | 3 | - | - | - | - | - | - |
| UNBEHANDELT | | 0 | 0 | 0 | 0 | 0 | 0 | 0 | 0 | 0 | 0 | 0 | 0 | 0 | 0 | 0 | 0 | 0 | 0 |



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

BEISPIEL 82

Auf Pinto-Bohnen wurden die Prüfsubstanzen in einem acetonhaltigen Lanolinöl gelöst appliziert. Die Applikation erfolgte, nachdem die zweiten Internodien eine Länge von 2 mm erreicht hatten. Es wurden 10 ug Wirkstoff aufgetragen. Die Auswertung erfolgte 4 Tage nach der Applikation. Das sich entwickelnde zweite Internodium wurde zum Teil sehr stark im Wachstum gehemmt.

| Erfindungsgemäße Verbindungen | Hemmung des Internodienwachstums in % |
|---|---|
| 1-Allyl-1-(4,6-dimethylpyrimidin-2-yl)-3-(2-methoxycarbonylphenylsulfonyl)-harnstoff | 40 |
| 1-Allyl-3-(2-chlorphenylsulfonyl)-1-(4,6-dimethoxy-1,3,5-triazin-2-yl)-harnstoff | 27 |
| 1-Allyl-1-(4,6-dimethoxy-1,3,5-triazin-2-yl)-3-(2-methoxycarbonylphenylsulfonyl)-harnstoff | 54 |
| 1-Allyl-3-(2-chlorphenylsulfonyl)-1-(4-methoxy-6-methylpyrimidin-2-yl)-harnstoff | 89 |
| 1-Allyl-3-(2-methoxycarbonylphenylsulfonyl)-1-(4-methoxy-6-methylpyrimidin-2-yl)-harnstoff | 43 |
| 1-(4,6-Dimethylpyrimidin-2-yl)-3-(2-äthoxycarbonylphenylsulfonyl)-1-propargyl-harnstoff | 42 |
| 1-Allyl-1-(4,6-dimethoxy-1,3,5-triazin-2-yl)-3-(2-äthoxycarbonylphenylsulfonyl)-harnstoff | 44 |
| 1-Allyl-1-(4-methoxy-6-methylpyrimidin-2-yl)-3-(2-äthoxycarbonylphenylsulfonyl)-harnstoff | 63 |
| 1-(4,6-Dimethylpyrimidin-2-yl)-3-(2-methoxycarbonylphenylsulfonyl)-1-propargylharnstoff | 75 |
| KONTROLLE | 0 |

-36-

## PATENTANSPRÜCHE

1. Substituierte Sulfonylharnstoffe der allgemeinen Formel

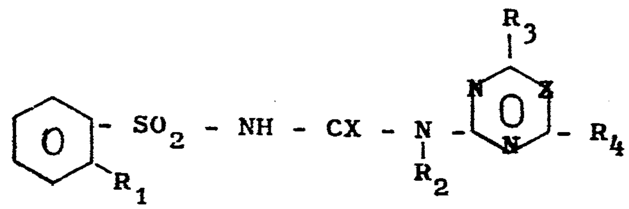

I

in der

$R_1$ Chlor, $-COOR_5$, $-S(O)_n-R_6$ oder 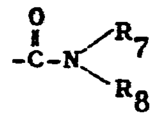 ,

$R_2$ die Gruppen 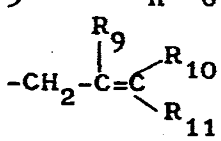 oder $-CH_2-C{\equiv}C-R_{11}$ ,

$R_3$ Wasserstoff, $C_1$-$C_4$-Alkyl, $C_1$-$C_4$-Alkoxy, $C_1$-$C_4$-Alkylthio, Halogen, Halogen-$C_1$-$C_4$-alkyl, Halogen-$C_1$-$C_4$-alkoxy, Di-$C_1$-$C_3$-alkyl-amino, $C_1$-$C_3$-Alkyl-amino oder $C_1$-$C_3$-Alkoxy-$C_1$-$C_3$-alkoxy,

$R_4$ Wasserstoff, $C_1$-$C_4$-Alkyl, $C_1$-$C_4$-Alkoxy, $C_1$-$C_4$-Alkylthio, Halogen, Halogen-$C_1$-$C_4$-alkyl, Halogen-$C_1$-$C_4$-alkoxy, Di-$C_1$-$C_3$-alkyl-amino, $C_1$-$C_3$-Alkyl-amino oder $C_1$-$C_3$-Alkoxy-$C_1$-$C_3$-alkoxy,

Z -CH= oder -N=,

$R_5$ $C_1$-$C_8$-Alkyl, $C_3$-$C_8$-Cycloalkyl, $C_1$-$C_3$-Alkoxy-$C_1$-$C_3$-alkyl, Phenyl, substituiertes Phenyl, Benzyl oder substituiertes Benzyl,

$R_6$ $C_1$-$C_6$-Alkyl oder Phenyl,

$R_7$ $C_1$-$C_4$-Alkyl,

$R_8$ $C_1$-$C_4$-Alkyl,

$R_7$ und $R_8$ $C_3$-$C_8$-Cycloalkyl, Morpholinyl, Pyrrolidinyl, Piperidyl oder Piperazinyl,

$R_9$ Wasserstoff, Chlor, Fluor oder $C_1$-$C_3$-Alkyl,

$R_{10}$ Wasserstoff, Chlor, Fluor, Trifluormethyl oder $C_1$-$C_3$-Alkyl,

$R_{11}$ Wasserstoff, Chlor, Fluor, Cyano, $C_1$-$C_4$-Alkyl oder Phenyl,

-37-

X Sauerstoff oder Schwefel und
n 0, 1 oder 2 bedeuten.

2. 1-Allyl-1-(4,6-dimethylpyrimidin-2-yl)-3-(2-methoxycarbonylphenylsulfonyl)-harnstoff.

3. 1-Allyl-3-(2-methoxycarbonylphenylsulfonyl)-1-(4-methoxy-6-methylpyrimidin-2-yl)-harnstoff.

4. 1-Allyl-1-(4,6-dimethoxy-1,3,5-triazin-2-yl)-3-(2-methoxycarbonylphenylsulfonyl)-harnstoff.

5. 1-Allyl-1-(4-methoxy-6-methylpyrimidin-2-yl)-3-(2-äthoxycarbonylphenylsulfonyl)-harnstoff.

6. 1-Allyl-3-(2-äthoxycarbonylphenylsulfonyl)-1-(4-methoxy-6-methyl-1,3,5-triazin-2-yl)-harnstoff.

7. 1-Allyl-1-(4,6-dimethoxy-1,3,5-triazin-2-yl)-3-(2-äthoxycarbonylphenylsulfonyl)-harnstoff.

8. 1-(4,6-Dimethylpyrimidin-2-yl)-3-(2-methoxycarbonylphenylsulfonyl)-1-propargyl-harnstoff.

9. 1-(4,6-Dimethylpyrimidin-2-yl)-3-(2-äthoxycarbonylphenylsulfonyl)-1-propargyl-harnstoff.

10. 1-Allyl-3-(2-chlorphenylsulfonyl)-1-(4-methoxy-6-methylpyrimidin-2-yl)-harnstoff.

11. 1-Allyl-3-(2-chlorphenylsulfonyl)-1-(4-methoxy-6-methyl-1,3,5-triazin-2-yl)-harnstoff.

12. 1-Allyl-3-(2-chlorphenylsulfonyl)-1-(4,6-dimethoxy-1,3,5-triazin-2-yl)-harnstoff.

13. Verfahren zur Herstellung von substituierten Sulfonylharnstoffen gemäß Ansprüchen 1 bis 12, dadurch gekennzeichnet, daß man

a) Verbindungen der allgemeinen Formel

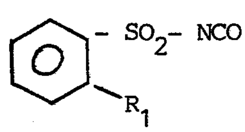

II

-38-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

mit Verbindungen der allgemeinen Formel

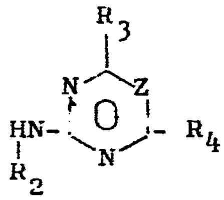

III

in Gegenwart eines inerten Lösungsmittels umsetzt, worin $R_1$, $R_2$, $R_3$, $R_4$ und Z die oben genannte Bedeutung haben.

14. Mittel mit herbizider und pflanzenwuchsregulierender Wirkung, gekennzeichnet durch einen Gehalt an mindestens einer Verbindung gemäß den Ansprüchen 1 bis 12.

15. Mittel gemäß Anspruch 14 zur Unkrautbekämpfung in Weizen-, Mais-, Reis-, Sojabohnen - und Baumwollkulturen.

16. Mittel gemäß Ansprüchen 14 und 15 in Mischung mit Träger- und/oder Hilfsstoffen.



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann · Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

0128274

Europäisches Patentamt

# EUROPÄISCHER RECHERCHENBERICHT

Nummer der Anmeldung

EP 84 10 2917

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 048 143 (E.I. DU PONT DE NEMOURS AND COMPANY) * Seiten 22,23 * | 1,14 | C 07 D 239/42<br>C 07 D 239/46<br>C 07 D 251/16<br>C 07 D 251/18<br>C 07 D 251/22<br>C 07 D 251/46<br>C 07 D 251/52<br>A 01 N 47/36 |
| | --- | | |
| A | NL-C- 121 788 (DEGUSSA) * Beispiel 1 * | 1,14 | |
| | --- | | |
| A | EP-A-0 030 433 (E.I. DU PONT DE NEMOURS AND COMPANY) * Patentansprüche * | 1,14 | |
| | ----- | | |
| | | | RECHERCHIERTE SACHGEBIETE (Int. Cl. 3) |
| | | | C 07 D 239/00<br>C 07 D 251/00 |

Der vorliegende Recherchenbericht wurde für alle Patentansprüche erstellt.

| Recherchenort | Abschlußdatum der Recherche | Prüfer |
|---|---|---|
| DEN HAAG | 12-09-1984 | VAN BIJLEN H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82